# EUROPEAN PATENT APPLICATION

(11) **EP 1 221 618 A1**
(43) Date of publication of application: **10.07.2002**
(21) Application number: 00126117.1
(22) Date of filing: 29.11.2000
(51) Int. Cl.: G01N 33/569, G01N 33/50, C12Q 1/68

(54) **Method for diagnosing allergic diseases**

(71) Applicant: GeneScan Europe AG, 79108 Freiburg (DE)
(72) Inventor: Schmidt-Weber, Carsten, 7260 Davos Dorf (CH); Blaser, Kurt, 7270 Davos Platz (CH); Wohlfahrt, Jan, 7270 Davos Platz (CH)
(74) Representative: Stürken, Joachim

(57) **Abstract**

mRNA of activated lymphocytes such as CD4⁺ T cells allows differential diagnosis of allergic diseases. The CD4⁺ T cells are isolated and stimulated under defined conditions in vitro. Subsequently, mRNA is subjected to multigene analysis such as DNA arrays. Expression profiling images, such as gene expression profiles, can be created, which allow on the basis of the activated T cell mRNA the prediction of certain phenotypes such as asthma or atopic dermatitis.

## Description

The present invention relates to methods for diagnosing allergic diseases and to screening assays for evaluating the allergenic and/or anti-allergical potential of analyte substances using expression profiling images of activated lymphocytes or monocytes/macrophages.

A list of the cited references with full bibliographic data can be found at the end of this specification.

Diagnosis of allergy and allergic asthma is of critical importance for disease management, research, epidemiology and development of specific therapies. Currently diagnosis of allergic diseases is limited to a number of proteins such as serum IgE which suggests but not proofs allergic reactions. Only the reaction against a specific antigen defines the presence of an allergic disease, making the IgE test to the most valuable test in allergy diagnosis (1, 2).

However the antigen is in many cases unknown, unless numerous and painful skin tests reveal an allergen. With serum IgE reacting against the allergen the definition for an allergic disease is fulfilled. But even the knowledge of an allergen does not allow conclusion of the immunological status of the patient or prediction of the future development of the disease. Diagnostic routine allows (in clinics with larger diagnosis facilities) the analysis of surface markers by FACS® routine, which is expensive and restricted to the analysis of only a few genes in parallel.

The design of new multigenic diagnostic systems is of critical importance for future management of multigenic diseases like allergy and asthma (3). These systems bear the possibility to: (A) define allergy with great reliability without knowing the antigen, (B) to define subclasses of the disease which allow better treatment and design of new (subgroup specific) drugs, (C) to define risk-groups for anaphylaxis or other complications, (D) to define patients which might be susceptible for immunotherapy, (E) to identify patients which will develop relative steroid resistance, (F) to identify the pathogenesis-causing genes and (G) to develop treatment strategies against these genes.

High throughput methods are now available which would allow the analysis of many expressed genes, however, it is not clear which cellular targets are suitable and which genes or gene products to be screened serve as reliable candidates for the desired purpose. For lung disease bronchial lavage fluids are reasonable targets which require invasive techniques not suitable for many physicians. In case of atopic dermatitis skin biopsies are performed, which allow only histological analysis with limited conclusions.

Blood is for diagnostic procedures the best choice since it can be collected with least invasive methods. The unseparated peripheral blood lymphocytes are in most cases subjected to variation of cell numbers even in healthy subjects and are therefore not of any help. T lymphocytes are known to be the cells, which react in the first line against the allergens and are therefore believed to induce disease, by helping B cells to produce IgE. However these cells were so far not been considered for diagnostic purposes, since the frequency of allergen specific in blood is very low (>0.1%; (4)). Even within skin lesions the frequency of a given allergen such as house dust mite is just 0.4% to 2.7% of all other T cells (5).

Therefore no attempt has been made so far to use, for example, the CD4⁺ T cell population, since changes of T cell gene expression are only expected in cells reacting against an allergen and not those which are allergen unreactive (>99% of the blood cells).

The use of T lymphocytes in proliferation assays allows the detection of a specific reaction of allergen reactive cells which is measurable following a week of in vitro cultivation (6, 7), and can be used to identify allergens of a given patient. These measurements, however, do not provide more information than any allergen-skin test, i.e. reactivity of a T cell against a given antigen.

Thus, an object of the invention is to provide a simple and reliable method for diagnosing allergic diseases and without knowing any specific allergen.

A further object of the present invention is to provide screening assays to evaluate the allergenic and/or anti-allergic potential of an analyte substance.

These and other objects and features of the invention will be apparent from the description, drawings, and claims, which follow.

The invention provides a method for diagnosing the presence of or predisposition for (a) allergic disease(s) using expression profiling images comprising the steps of:
(a) *in vitro* activating lymphocytes or monocytes/macrophages obtained from whole blood of an individual to be diagnosed for the presence of or predisposition for (a) specific allergic disease(s),
(b) providing ligands selected from the group consisting of mRNA, oligonucleotides, cDNAs, proteins or functional fragments thereof, isolated or generated from said lymphocytes or monocytes/macrophages,
(c) contacting said ligands with a set of immobilized receptors selected from the group consisting of oligonucleotide or cDNA probes and antibodies or functional fragments thereof, specifically representing a respective set of genes involved with the presence of or predisposition for (a) specific allergic disease(s) to be diagnosed, and qualitatively and quantitatively detecting the presence of bound ligand/receptor complexes to obtain an expression profiling image being representative for the current status of the individual to be diagnosed,
(d) comparing the expression profiling image obtained in step (c) with the expression profiling image(s) of normal individuals and/or with the expression profiling images(s) of individuals having a predisposition for or suffering from (a) specific allergic disease(s) to be diagnosed, and
(e) excluding or diagnosing (a) suspected allergic disease(s) or a predisposition therefor on the basis of the results of the comparison obtained in step (d).

An advantage of the inventive method is that differential diagnosis of different allergic diseases is possible on the basis of the respective different expression profiling images in vitro, i.e. outside the body. Moreover, in a further embodiment of the inventive method usefull as a screening assay potential allergenic substances or anti-allergical substances may be identified.

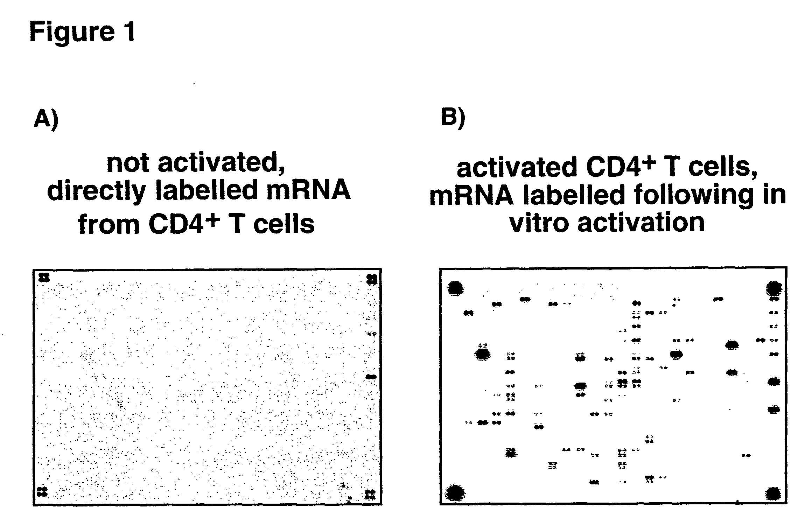

The step of in vitro activating the cells is important. When for example in step (c) as contacting hybridizations were performed without in vitro activation of the cells, signals, e.g. on an array, were inconsistent in quality or even absent except of a few housekeeping genes (Fig. 1a, housekeeping genes on the right side). This effect possibly reflects different activation conditions of individual patients. In contrast, in vitro activation enhanced signals on the array, for example a membrane, surprisingly also of genes which are not regulated by anti-CD3/CD28 such as integrins. It appears that anti-CD3/CD28 stimulation enhances the allover transcription machinery, resulting in more mRNA transcripts. The T cell stimulation step therefore contributes to the normalization of the activation status of the cells and in turn for the standardization of the diagnostic procedure which is important for intra-patient comparisons.

Further advantageous and/or preferred embodiments of the invention are subject-matter of the further independent claims and the subclaims.

In one embidiment of the invention in step (a) of the method said lymphocytes are selected from the group consisting of CD4⁺ T cells and CD8⁺ T cells. Using CD8⁺ T cells may be preferred, in particular when lung diseases are initiated by viral infections, or monocytes might be used for patients with additional inflammatory diseases. It should further be noted that the activation or stimulation in step (a) of the inventive method can also be performed in full, unseparated blood prior to the separation of the cells. The latter modification allows automatic procedures, which will be critical for controlling the costs of the diagnostic procedure.

In a further embidiment of the invention in step (a) of the method said lymphocytes or monocytes/macrophages are activated by exposing the cells to mitogens such as anti-CD3 and/or anti-CD28 antibodies; or phorbol 12-myristate 13-acetate and ionomycin; or phytohemagglutinin and ionomycin; or staphylococcal enterotoxin B; or lipopolysaccharid; or a combination thereof. The anti-CD3 and/or anti-CD28 antibodies may be, for example, monoclonal antibodies, which optionally may be plate-bound or crosslinked.

In yet a further embodiment of the invention in step (b) of the method the ligand is for example labelled with one or more label(s) selected from the group consisting of colored, fluorescent, bioluminescent, chemoluminescent, phosporescent or radioactive labels, an enzyme, an antibody or a functional fragment or derivative thereof, a protein A/gold based system, a biotin/avidin/streptavidin based system or an enzyme electrode based system is/are used.

In a preferred embodiment of the inventive method the allergic disease(s) or predisposition(s) to be diagnosed is/are selected from the group consisting of allergic asthma, allergic rhinitis, food allergies, anaphylactic shock risk, atopic dermatitis, immediate-type allergic reactions, and insect allergies.

More preferrably the allergic disease(s) to be diagnosed is/are allergic asthma and atopic dermatitis. By excluding allergic asthma it is possible to diagnose non-allergic asthma, which differential diagnosis is of clinical importance.

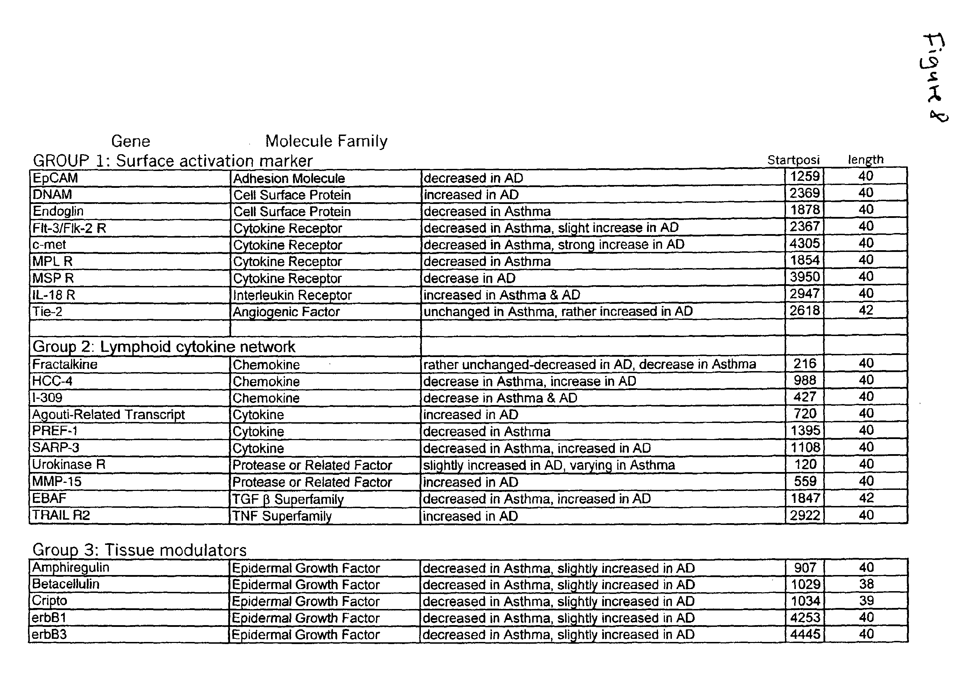

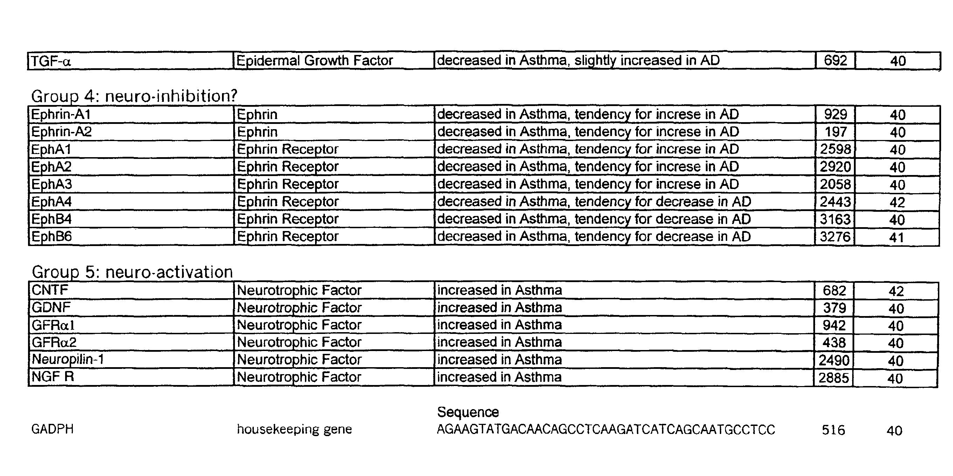

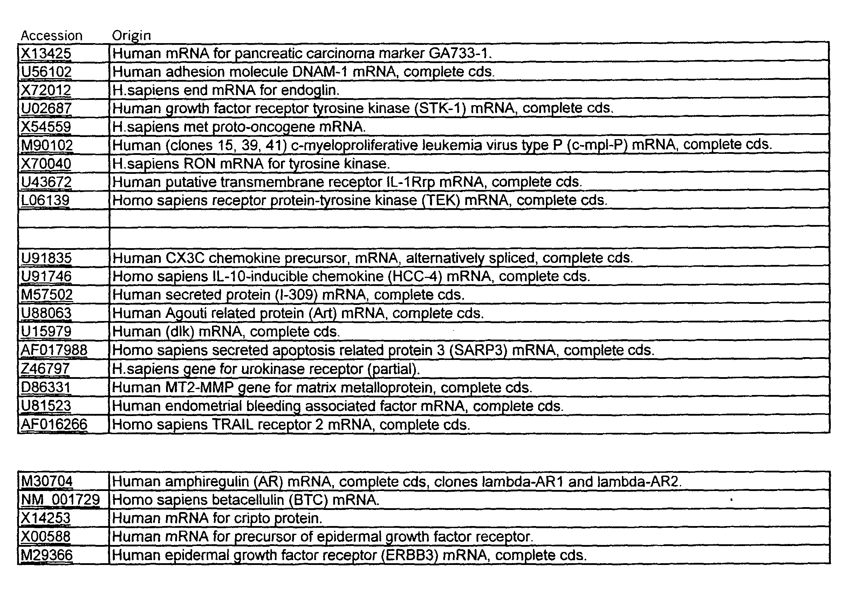

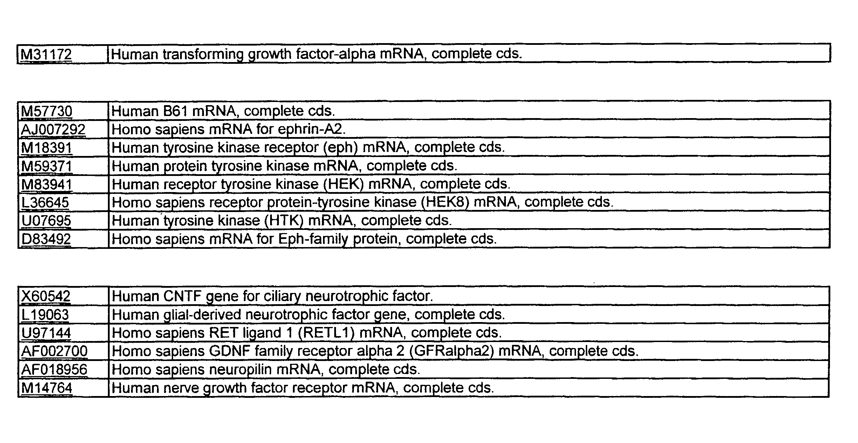

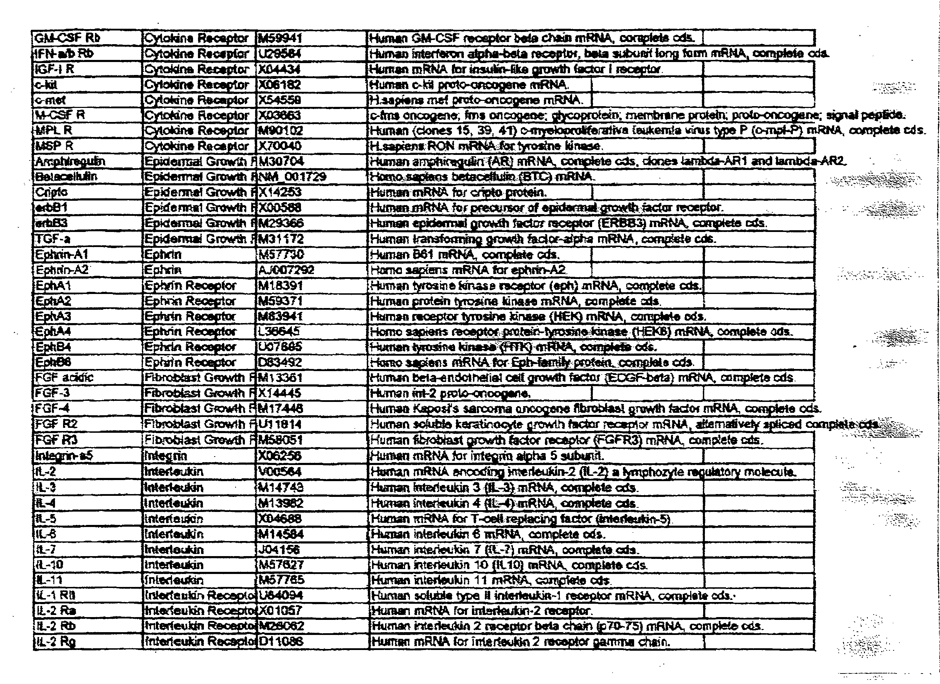

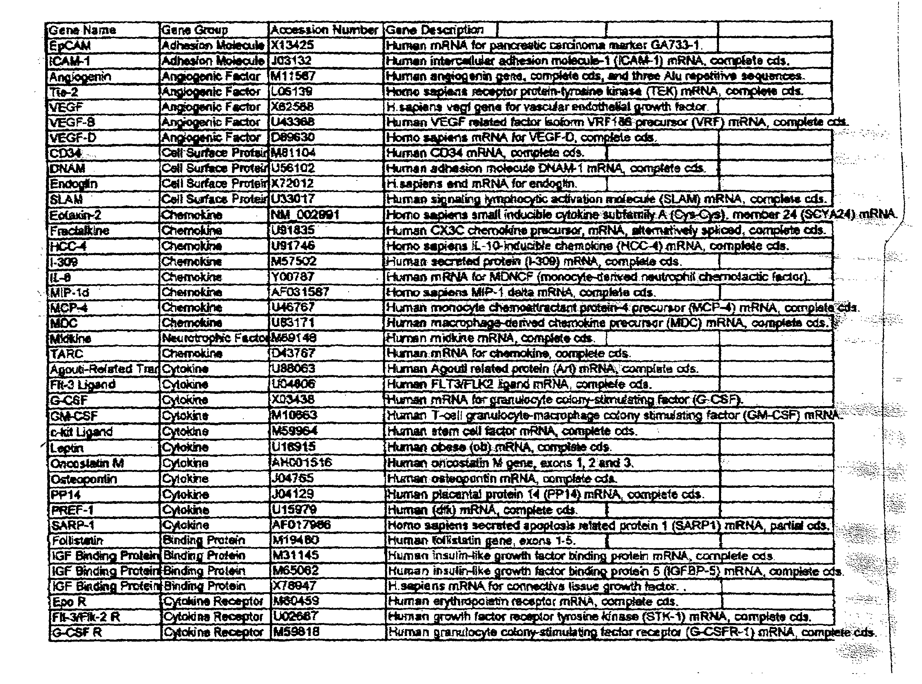

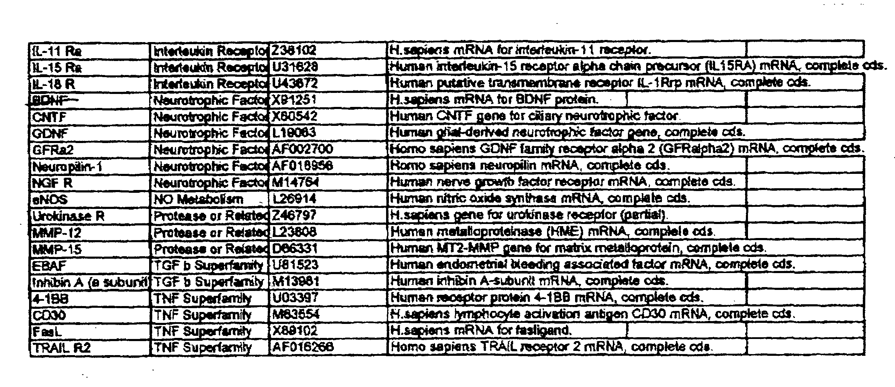

For diagnostic purpose, in step (c) of the inventive method said set of receptors specifically representing a respective set of genes is selected from the group consisting of genes coding for EpCAM, DNAM, Endoglin, Flt-3/Flk-2 R, c-met, MPL R, MSP R, IL-18 R, Tie-2, fractalkine, HCC-4, I-309, Agouti-related transcript, PREF-1, SARP-3, Urokinase R, MMP-15, EBAF, TRAIL R2, amphiregulin, betacellulin, cripto, erbB1, erbB3, TGF-α, ephrin-A1, ephrin-A2, EphA1, EphA2, EphA3, EphA4, EphB4, EphB6, CNTF, GDNF, GFRα1, GFRα2, neurophilin-1, NGF R, ICAM-1, angiogenin, VEGF, VEGF-8, VEGF-D, CD34, SLAM, eolakin-2, IL-8, MIP-1d, MCP-4, MDC, midkine, TARC, Flt-3 ligand, G-CSF, GM-CSF, c-kit ligand, leptin, oncostatin M, osteopontin, PP14, SARP-1, follistatin, IGF binding protein, IGF binding protein, IGF binding protein, Epo R, G-CSF-R, IL-11 Ra, IL-15 Ra, eNOS, MMP-12, inhibin A (a subunit), 4-1BB, CD30, FasL, TRAIL R2, GM-CSF Rb, IFN-a/b Rb, IGF-I R, c-kit, M-CSF R, FGF acidic, FGF-3, FGF-4, FGF R2, FGF R3, integrin-a5, IL-2, IL-3, IL-4, IL-5, IL-6, IL-7, IL-10, IL-11, IL-1 RII, IL-2 Ra, IL-2 Rb, IL-2Rg; and allelic variants or mutants thereof. In Figure 8 these genes are roughly classified into 5 groups. For the preparation of a statistically significant normal or allergic expression profiling image 3 to 5 genes from each group may be sufficient. It might, however, be possible that a specific allergic disease requires more than 5 genes.

Preferably, said set of receptors is selected from oligonucleotide probes having a sequence defined by, or correlated to, or derived from the group of genes consisting of candidate genes indicated in the following list:

| Gene coding for | Startposition | lenght | Accession No |
|---|---|---|---|
| EpCAM | 1259 | 40 | X13425 |
| DNAM | 2369 | 40 | U56102 |
| Endoglin. | 1878 | 40 | X72012 |
| Flt-3/Flk-2 R | 2367 | 40 | U02687 |
| c-met | 4305 | 40 | X54559 |
| MPL R | 1854 | 40 | M90102 |
| MSP R | 3950 | 40 | X70040 |
| IL-18 R | 2947 | 40 | U43672 |
| Tie-2 | 2618 | 42 | L06139 |
| Fractalkine | 216 | 40 | U91835 |
| HCC-4 | 988 | 40 | U91746 |
| I-309 | 427 | 40 | M57502 |
| Agouti-Related Transcript | 720 | 40 | U88063 |
| PREF-1 | 1395 | 40 | U15979 |
| SARP-3 | 1108 | 40 | AF017988 |
| Urokinase R | 120 | 40 | Z46797 |
| MMP-15 | 559 | 40 | D86331 |
| EBAF | 1847 | 42 | U81523 |
| TRAIL R2 | 2922 | 40 | AF016266 |
| Amphiregulin | 907 | 40 | M30704 |
| Betacellulin | 1029 | 38 | NM 001729 |
| Cripto | 1034 | 39 | X14253 |
| erbB1 | 4253 | 40 | X00588 |
| erbB3 | 4445 | 40 | M29366 |
| TGF-a | 692 | 40 | M31172 |
| Ephrin-A1 | 929 | 40 | M57730 |
| Ephrin-A2 | 197 | 40 | AJ007292 |
| EphA1 | 2598 | 40 | M18391 |
| EphA2 | 2920 | 40 | M59371 |
| EphA3 | 2058 | 40 | M83941 |
| EphA4 | 2443 | 42 | L36645 |
| EphB4 | 3163 | 40 | U07695 |
| EphB6 | 3276 | 41 | D83492 |
| CNTF | 682 | 42 | X60542 |
| GDNF | 379 | 40 | L19063 |
| GFRalpha1 | 942 | 40 | U97144 |
| GFRalpha2 | 438 | 40 | AF002700 |
| Neuropilin-1 | 2490 | 40 | AF018956 |
| NGF R | 2885 | 40 | M14764 |

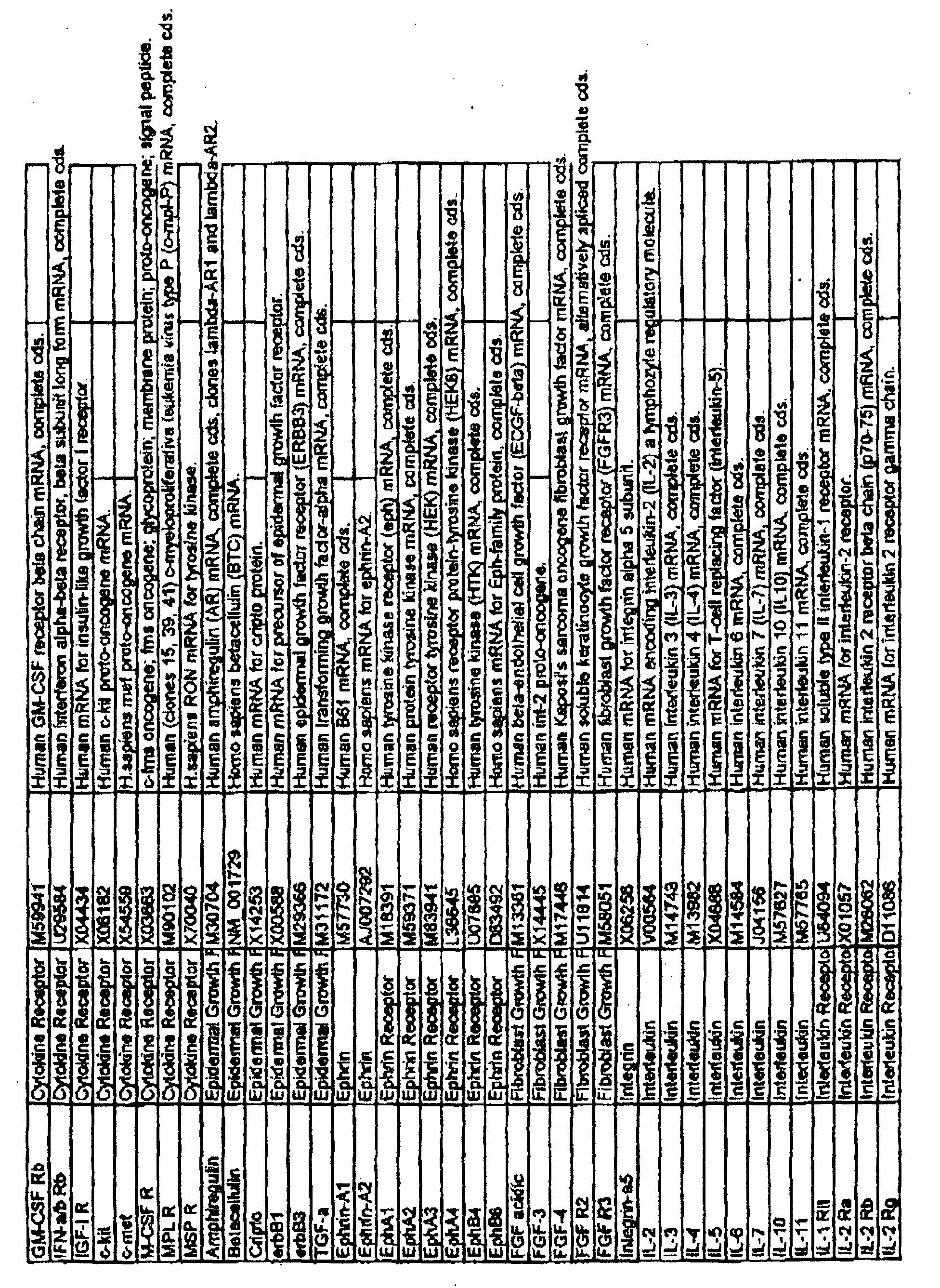

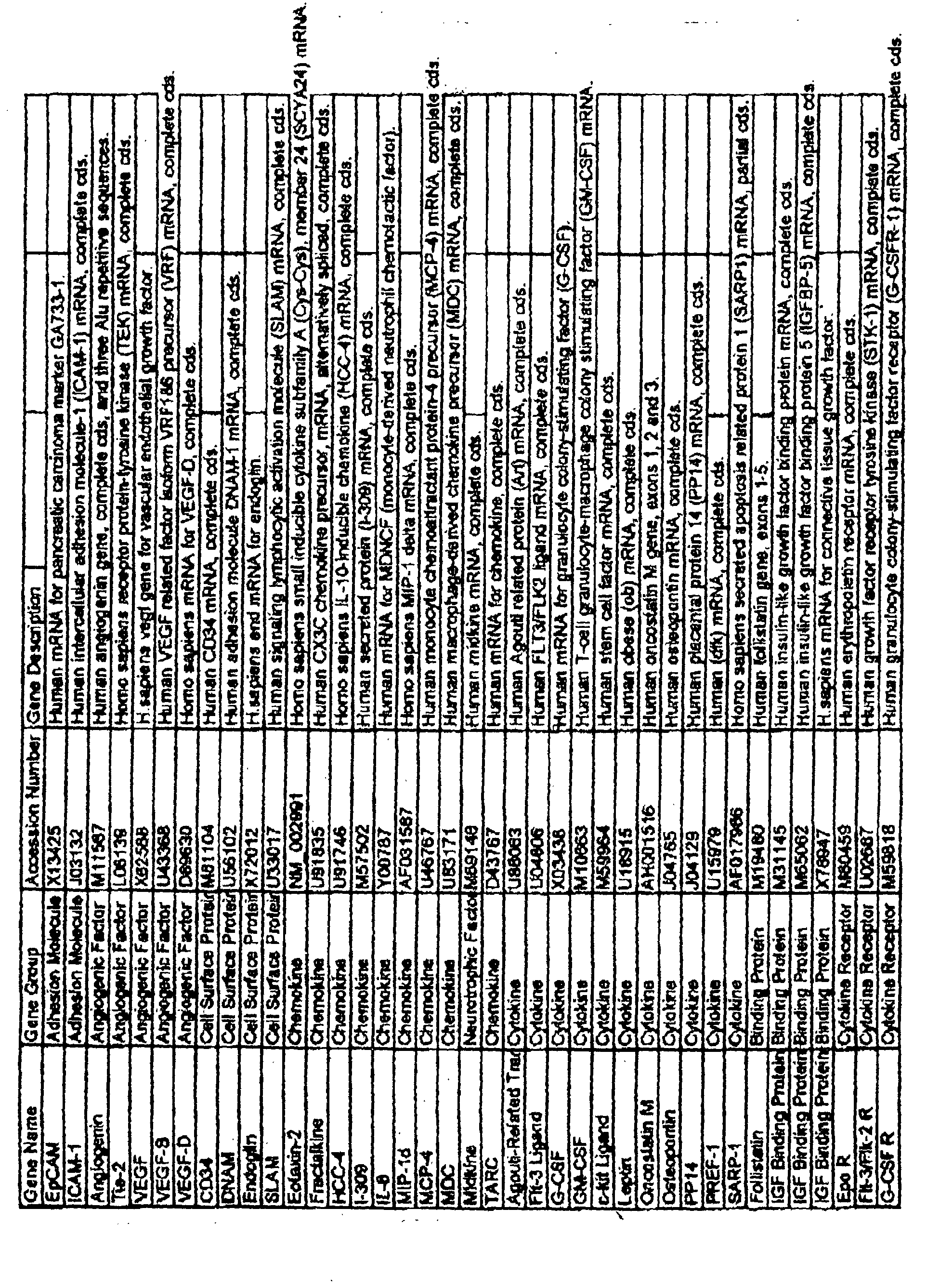

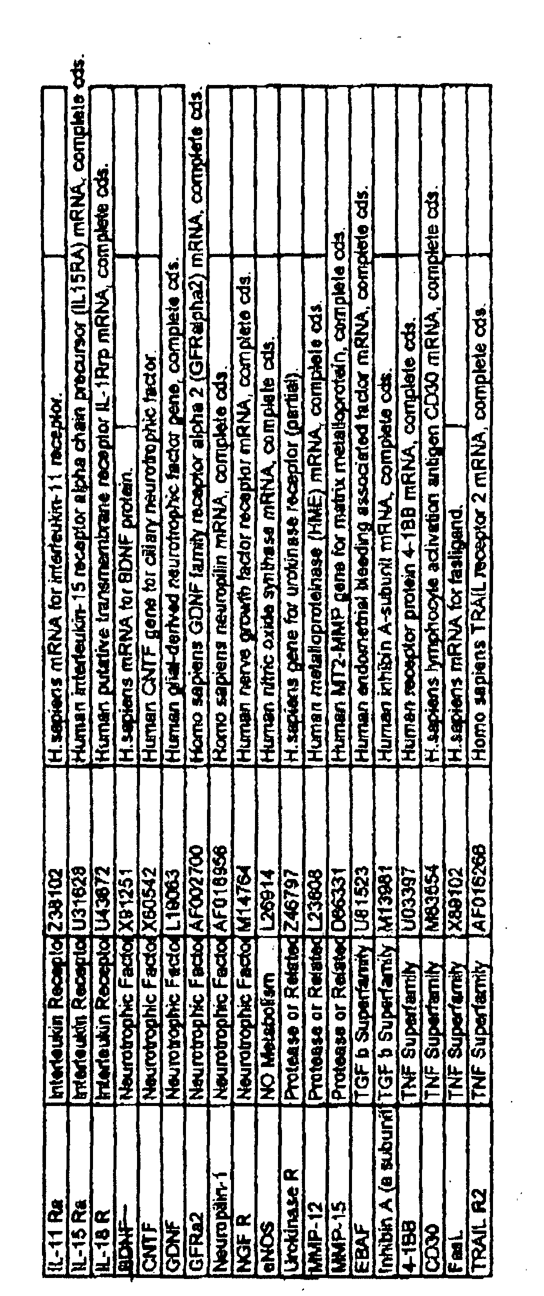

, wherein each oligonucleotide probe is obtainable on the basis of structural information mediated by the sequence data provided by the respective accession number set out for each candidate gene.

For example, suitable sequences which may be used for oligonucleotide design may be downloaded from the NCBI GenBank (http://www.ncbi.nlm.gov/GenBank/index.html) using the Accession number listed in Figure 8. All accession numbers give access to mRNA sequences; since the cDNA of the patients is reverse transcribed, the oligonucleotide have to sense strand and are therefore identical with the mRNA sequence. The oligonucleotide position (see Figure 8) is selected using the Vector NTI 4.0 software (Informix, Gaitherburg, MD, USA) using following settings for the search of hybridization probes: size of oligonucleotide probe 40 ± 2; melting temperature of Tm = 75 ± 3 °C and a GC content of 45-60%.

In another embodiment of the invention in step (c) of the method said immobilized receptors are located on a surface, and may optionally form a patterned array thereon.

In a further aspect the invention provides a screening assay for evaluating the allergenic and/or anti-allergical potential of (an) analyte substance(s) using expression profiling images comprising the steps of:
(a) in vitro activating lymphocytes or monocytes/macrophages, preferably being standardized to represent a baseline expression profiling image characteristic for a normal's status,
(b) incubating said activated lymphocytes or monocytes/macrophages with (an) analyte substance(s) to be evaluated for its (their) allergenic and/or anti-allergical potential,
(c) providing ligands selected from the group consisting of mRNA, oligonucleotides, cDNAs, proteins or functional fragments thereof, isolated or generated from said lymphocytes or monocytes/macrophages,
(d) contacting said ligands with a set of immobilized receptors selected from the group consisting of oligonucleotide or cDNA probes and antibodies or functional fragments thereof, specifically representing a respective set of genes involved with the presence of or predisposition for (a) specific allergic disease(s) to be diagnosed, and qualitatively and quantitatively detecting the presence of bound ligand/receptor-complexes to obtain an expression profiling image being representative for the allergenic and/or anti-allergical potential of the analyte substance(s) to be evaluated,
(e) evaluating the allergenic and/or anti-allergical potential of the analyte substance(s) by comparing the expression profiling image obtained in step (d) with the expression profiling image generated from normal individuals, or preferably by directly analyzing the expression profiling image obtained in step (d).

The "risk degree" of an allergenic substance can be concluded by comparison of the expression profiles with substances of known risk (such as diesel exaust, Aspergillus allergens etc.).

For example, food allergies, anaphylactic shock risk patients, immediate-type allergic reactions, insect allergies, allergies against environmental pollutants or mediaments may be easily detected.

The current allergic status of a patient, for example treated with an anti-allergically active substance, may be monitored easily with the inventive screening assay. This allows a prompt adaptation of the treating regime (for example dosing, type of administration) to the current allergic status. Antiallergic substances are for example sympathomimetic drugs, histamine antagonists, glucocorticoids, and corticosteroids. Specific examples may be found in any standard textbook on pharmacology, cf. for example Goodman & Gilman's, "The Pharmacological Basis of Therapeutics", Vols and 2, 8^{th} edition, McGraw-Hill International Editions, 1992, pages 217, 587, 1455 and 1575/76 the disclosure content of which is herein incorporated by reference.

Of particular clinical importance is that patients with relative steroid resistance or patients that will develop such resistance may be identified easily with the inventive screening assay.

In another aspect the invention provides a diagnostic tool suitable for use in the inventive methods comprising on its surface a set of immobilized receptors selected from the group consisting of oligonucleotide or cDNA probes and antibodies or functional fragments thereof, capable of specifically binding ligands selected from the group consisting of mRNA, oligonucleotides, cDNAs, proteins or functional fragments thereof, representing a respective set of genes involved with (a) specific allergic disease(s) or a predispositon therefor to be diagnosed. Optionally, said immobilized receptors may form a patterned array on the surface. If, for example fluorescent nucleotides are used the surface may be made of glass or any other solid, non-fluorescent carrier.

The inventive method generally allows the generation of characteristic expression profiling images for diagnosing allergic diseases based both on genes and the respective gene products, i.e. proteins. In a specific embodiment gene expression profiling images or gene expression profiles are used.

As already mentioned above, the present invention proposes the use of mRNA isolated from for example CD4⁺ T cells or monocytes/macrophages activated in vitro, for example for 6h, as a basis for multigene analysis for differential diagnosis of allergic and asthmatic diseases. Here it is demonstrated for the first time that, for example, hybridization techniques are sensitive enough to profile T cell or monocytes/macrophages gene expression and that multigene approaches allow on the basis of activated CD4⁺ T cell or monocytes/macrophages mRNA the differentiation between clinically different allergic diseases, such as allergic asthma and atopic dermatitis by gene expression profiles.

In the following the invention is described in more detail, however, without any limitation, with respect to complementary nucleic acids or fragments thereof as ligand/receptors pairs.

In practicing the invention, after steps (a) and (b) of the inventive method each gene, i.e. labelled cDNA derived therefrom, involved with the presence of or predisposition for (a) specific allergic disease(s) to be diagnosed is spotted in duplicates onto, for example, an array membrane with respective immobilized oligonucleotide probes capable of specifically hybridizing with the respective gene, i.e. labelled cDNA derived therefrom, and the mean of both measurements is used in subsequent evaluations. Differences in gene expression are accepted as different when the signals from patient material are at least two fold lower or higher than those from healthy volunteers. This threshold is commonly accepted for the evaluation of expression arrays. All genes are corrected against housekeeping genes to compensate minor differences in cell load of each sample.

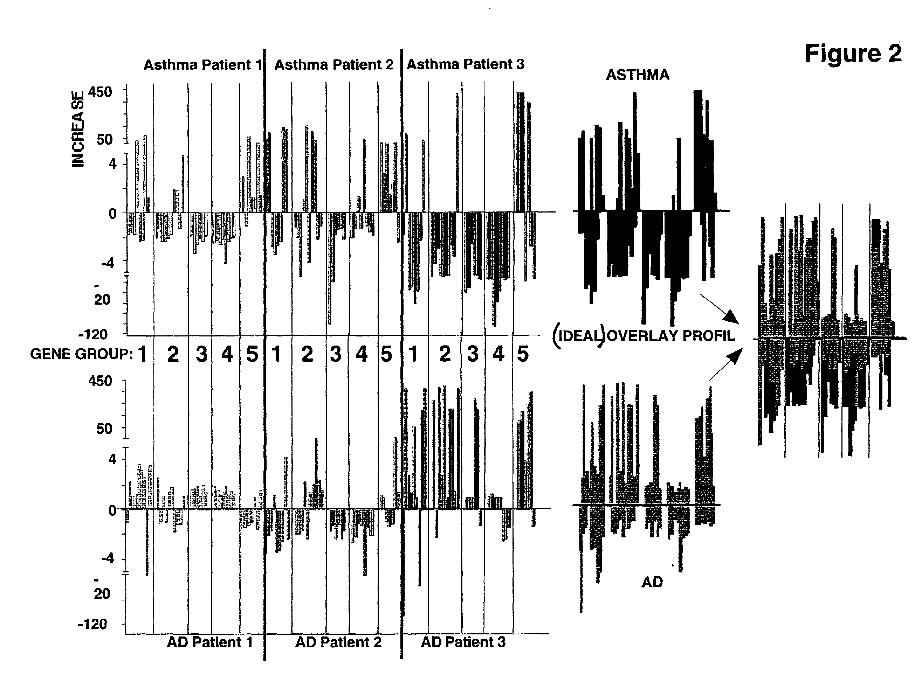

Fold differences are calculated for each gene and since the order and scale of each patient is the same an individual profile for each patient results. These profiles are now overlaid (middle panel of Figure 2), for example for all atopic dermatitis and for all asthma patients, resulting in an (ideal) asthma or atopic dermatitis profile. This profile is defined by the probability value *p*, which will be defined for each individual gene or gene groups in correlation (Spearman Rank Test) with clinical parameters of asthma or atopic dermatitis. For normal profiles healthy volunteers are used.

The inventive method is in addition supported by statistical analysis, which defines statistical confidence intervals, which link for example a 5 fold decrease in gene x with 10% confidence to phenotype y; a 7 fold decrease in gene x with 50% confidence to phenotype y. Again this confidence is defined on the basis of probability value P (precise formula given in (8)). Even if statistical correlation with a given phenotype is relatively low using the inventive method, the practically unlimited number of genes and their statistical confidence will be in sum 99%, since the p values are added for the confidence intervals (8). The number of genes necessary for the method of the invention depends finally on the phenotype to be described and its extend of correlation with each gene. For example 3 to 5 genes from each group in the table of Figure 8 may be sufficient to obtain a statistically significant normal or allergic profile.

The foregoing and other objects of the invention, the various features thereof, as well as the invention itself, may be more fully understood from the following description, when read together with the accompanying drawings, in which:

Figure 1 shows autoradiographs of labelled mRNA from not activated and activated CD4+ cells.

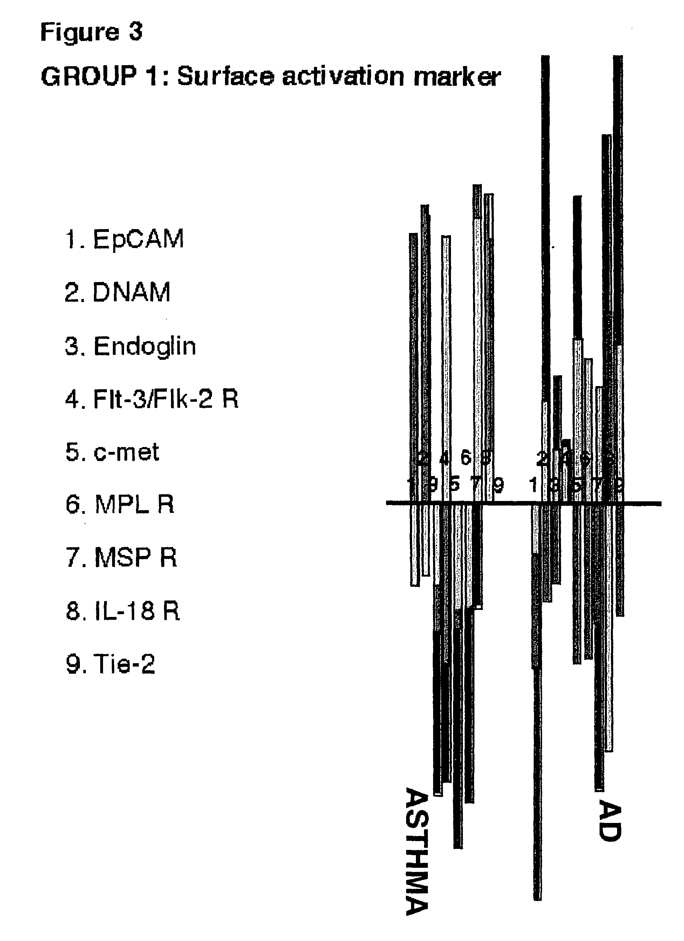

Figures 2 shows gene expression profiles of individual patients suffering from allergic asthma or atopic dermatitis. Three blocks are representing three individual patients. Of note these bars represent changes in comparison to healthy volunteers. Within each patient block are 5 groups which contain genes which share certain functions. Each group is depicted separately in Figure 3-7 including the gene designation. On the right next to the individual patients are overlays of the patient groups shown. These overlays demonstrate that some genes are exclusive marker for atopic dermatitis or asthma respectively whereas other genes are "misregulated" both in atopic dermatitis and asthma.

Figure 3 shows surface genes (group 1) which are likely to be expressed during the T cell activation process. The left panel shows the changes in atopic dermatitis patients (AD) and the right panel the asthma patients. The bars represent changes in fold expression. A bar upward from the black lines indicates an increase a bar downwards a decrease of expression. Epcam (lane 1) was in all three AD patients decreased, whereas asthma patients show either a weak decrease or even an increased expression. DNAM (lane 2) was increased in AD patients except in one patient, similarly to asthma patients, possibly indicating a common allergy gene. Endoglin (lane 3) was decreased in all asthma patients whereas AD patients showed increased expression in two cases and only a minor decrease in one case. The ladder patient is possibly predisposed for developing asthma. Flt-3/Flk-2 R (4) was always found to be increased in AD patients whereas asthma patients showed decreased expression except in one case. C-met (lane 5) was again decreased in all asthma patients and increased in AD patients with exception of the putative "asthma predisposed" patient. MPL R (lane 6) behaved as described for C-met. MSP-R (lane 7) was dramatically increased in two asthma patients with the exception of one patient who showed a minor decrease, in contrast two AD patients showed a decrease in gene expressionand only one patient showed a minor increase. The expression of the IL-18-R (lane 8) was more heterogeneously expressed, however increases observed both in asthma and also a single but dramatic decrease in an AD was observed. Tie-2 (lane 9) was unchanged in asthma patients but dramatically up regulated in two AD patients and rather to a minor degree decreased in another AD patient.

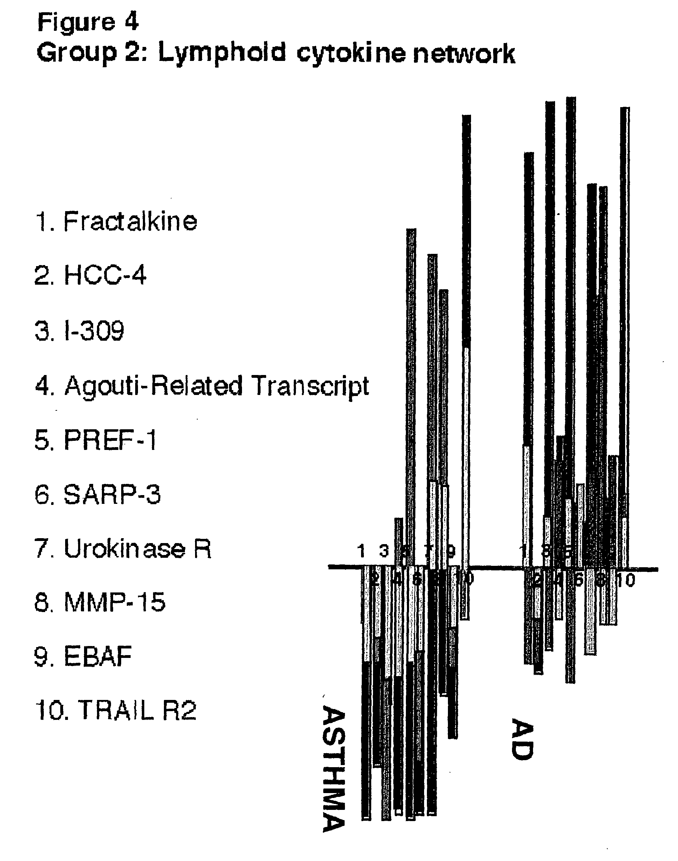

Figure 4 shows cytokines (group 2) which are important for cross talk between lymphocytes and other cells. The left panel shows the changes in atopic dermatitis patients (AD) and the right panel the asthma patients. The bars represent changes in fold expression. A bar upward from the black lines indicates an increase a bar downwards a decrease of expression. Fractalkine (lane 1) was always found to be decreased in asthma patients and increased in two out of three AD patients. The AD patient which showed a decrease is identical with the putative "asthma predisposed" AD patient described in Figure 3. HCC-4 (was decreased in all asthma and all AD patients, possibly representing a common allergy marker, which is known to be regulated by IL-10, a suppressive cytokine. I-309 (lane 3) beahves as described for Fractalkine. The Agouti-related transcript was decreased in two and increased to a minor degree in one asthma patient: in AD patients changes occured in a lower amplitude but reverse (increased) in comparison to the asthma patients. PREF-1 (lane 5) showed a dramatic increase in a single asthma patient and a decrease in the other two patients: in AD patients this gene behaved as described for Fractalkine. SARP-3 (lane 6) decreased in all asthma patients whereas expression was unchanged in AD patients except of one case where a minor increase was observed. The urokinase receptor was mostly upregulated both in asthma and AD, indicating an allergy related misregulation of this gene. MMP-15 (lane 8) expression is comparable with the urokinase receptor described above.

EBAF (lane 9) showed only minor changes in AD patients, whereas downregulation was detected in all three asthma patients. TRAIL R2 (lane 10) was upregulated in all three AD patients and also in two asthma patients. Only one asthma patient showed a minor decrease in TRAIL R2 expression.

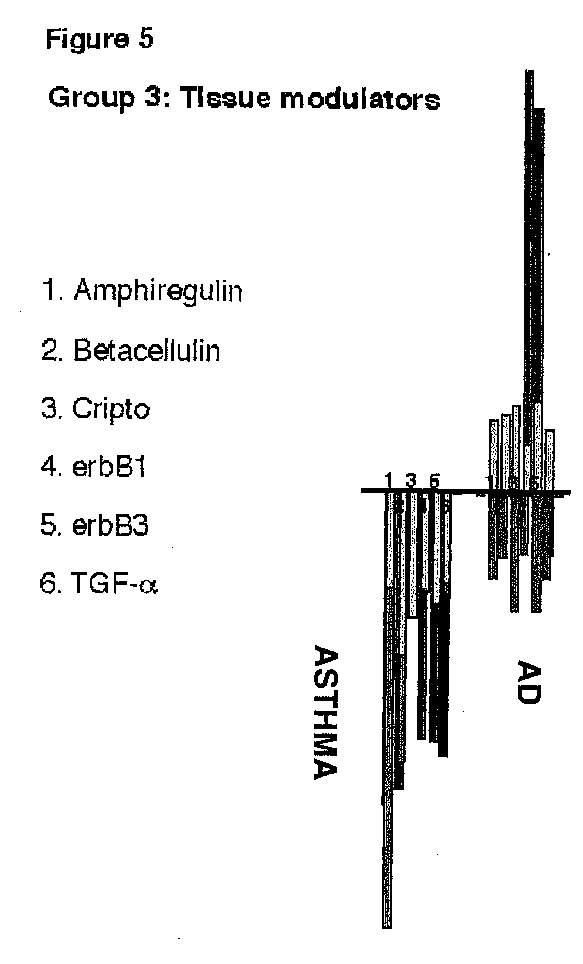

Figure 5 shows factors important for tissue modeling (group 3). The left panel shows the changes in atopic dermatitis patients (AD) and the right panel the asthma patients. The bars represent changes in fold expression. A bar upward from the black lines indicates an increase a bar downwards a decrease of expression. Amphiregulin (lane 1) was downregulated in all three asthma patients. A mild increase in Amphiregulin expression was only observed in one AD patient and a decrease in the patient described above with the putative "asthma predisposition". Betacellulin (lane 2) showed the same patterns as Amphiregulin. Cripto (lane 3) showed a decrease in two asthma patients and behaved for the AD patients as described for the preceding genes. ErbB1 (lane 4), ErbB3 (lane 5) and TGF-alpha (lane 6) were in all three asthma patients decreased and increased (in particular ErbB1 & 3) in the AD patients with the exception of the putative "asthma predisposition" patient.

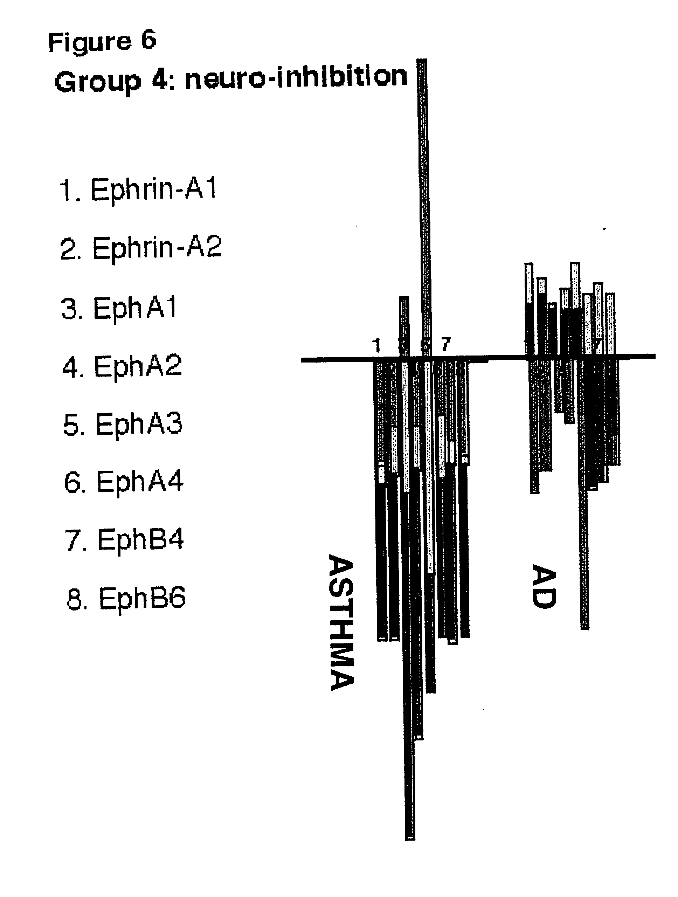

Figure 6 shows factors (group 4) related to neurological biology which are however not yet functionally described. The left panel shows the changes in atopic dermatitis patients (AD) and the right panel the asthma patients. The bars represent changes in fold expression. A bar upward from the black lines indicates an increase a bar downwards a decrease of expression. Strikingly, all of the genes (lanes 1-8; Ephrin-A1, Ephrin-A2, EphA1-4 and EphB4 & 6) were decreased in all asthma patients with the exception EphA1 (lane 3) and EpHA3 (lane 5), which was increased in one asthma patient. As in previous figures, the putative "asthma predisposition" patient showed also a decrease in all genes. In contrast, Ephrin-A1, A2 and EphA1-3 (lanes 1-5) were increased in all other AD patients. EphA4, B4 and B6 (lanes 6-8) were decreased in one AD patient and decreased in the third one.

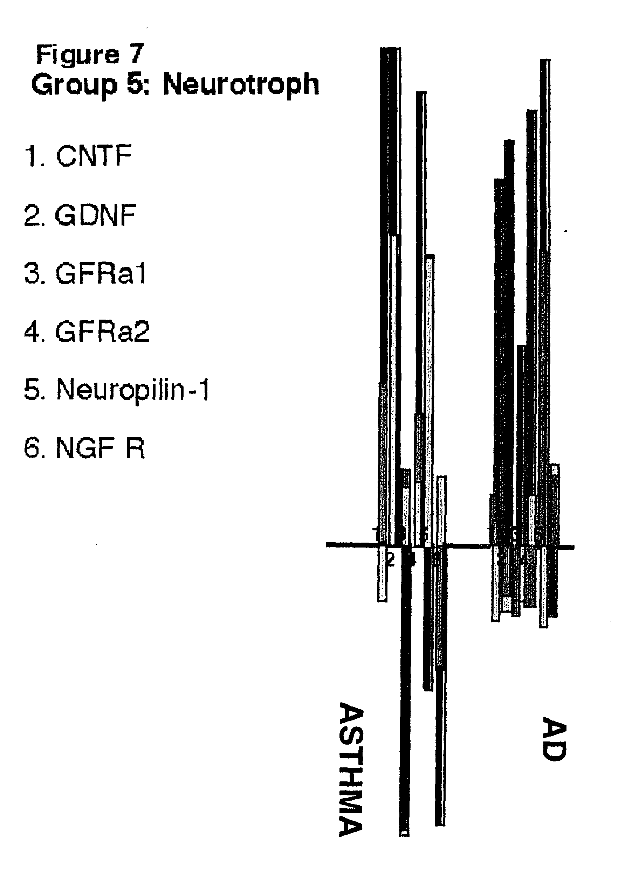

Figure 7 shows neurotrophic factors (group 5) which allow the T cells the communication with neuronal cells. The left panel shows the changes in atopic dermatitis patients (AD) and the right panel the asthma patients. The bars represent changes in fold expression. A bar upward from the black lines indicates an increase a bar downwards a decrease of expression. CNTF (lane 1) was increased in two patients decreased to a minor degree in one AD patient. In Asthma two patients with strong increase and one patient with a minor decrease were observed. GDNF (lane 2) was increased in one patient and decreased in two AD patients however the greater amplitude was seen in the case of increased expression. Also asthma patients showed only increased expression of this gene with a great amplitude. GFRa1 (lane 3) was increased and decreased in both disease, but the greatest amplitude was seen in the increased expression of AD patients and the greatest in decrease in the asthma patient. GFRa2 (lane 4) behaved as BDNF in AD patients, whereas all three asthma patients showed an increase in gene expression. Neuropilin-1 (lane 5) was dramatically increased in two AD patients and decreased to a minor degree in another AD patient. Among asthma patients Neuropilin was increased in one patient and decreased in another patient and unchanged in a third one. NGF R (lane 6) Increased in two AD patients to a minor degree and decreased to the same extend in another one. Although one asthma patient was observed with a mild increase in NGF R expression, the two other patients showed a marked decrease in NGF R expression. Of not, the AD patient with "asthma predisposition" was not observed to be asthma-like in this panel, indicating that genes of this group might correlate with acute clinical sympthoms.

Figure 8 shows a table listing genes involved with allergic asthma or atopic dermatitis (AD). The genes are clustered as described in figures 3-7. Further, positions of sequences for oligonucleotide probes (with data bank accession numbers for the respective genes) are given. The oligonucleotides in a size of 40 ± 2 base pairs were selected on the basis of a common melting temperature (Tm 75 ± 3 °C, GC content: 45-60%).

In the following the invention is disclosed in more detail with reference to examples and to drawings. However, the described specific forms or preferred embodiments are to be considered in all respects as illustrative and not restrictive, the scope of the invention being indicated by the appended claims rather than by the following description, and all changes which come within the meaning and range of equivalency of the claims are therefore intended to be embraced therein.

Particularly, it should be appreciated that in the following description activated CD4⁺ T cells are used only as a specific example for suitable blood cells, and that allergic asthma and atopic dermatitis are only specific examples for allergic diseases.

### Examples

To perform diagnosis tests 80 ml blood is taken from the patient using vacuum container containing Heparin (Vacutauner™, Becton Dickinson, Pymouth, UK). For pediatric tests the volume of blood can be downscaled to 5 ml if the RNA is amplified following isolation and prior labelling. CD4⁺ T cells are separated from other blood cells using either Ficoll (Biocoll separating solution, Biochrom KG, Berlin, Germany) density centrifugation following the instructions of the manufacturer. Purified peripheral blood mononuclear cells (PBMC) are subsequently purified using the anti-CD4 coated magnetic beads (DetachaBead, Dynal, Oslo, Norway). The purity of the isolated population is verified by FACS® analysis and is always higher than 95%.

Cells are cultured overnight with plate-bound anti-CD3 (1µg / ml; OKT3 clone from ATCC, Manassas, VA, USA) and anti-CD28 (2µg / ml; 15E8 from CLB, Amsterdam, The Netherlands) monoclonal antibodies. All cultures are carried out in RPMI 1640 supplemented with 1 mM L-glutamine, sodium pyruvate, non-essential amino acids, 5 x 10⁻⁵ µM 2-mercaptoethanol and 10% heat-inactivated fetal calf serum (all from Life Technologies, Basel, Switzerland).

For RNA analysis 5 x 10⁶ cells of each cell population are lysed with lysis buffer (Rneasy, Qiagen, Hamburg, Germany). Alternatively cells can be directly purified from blood by using the Dynal beads described above, avoiding the Ficoll step. There is preliminary evidence that stimulation with plate-bound antibodies can be replaced by mitogenic stimulation with phorbol 12-myristate 13-acetate (PMA) and ionomycin or phytohemagglutinin (PHA) and ionomycin. As already mentioned above, this stimulation can also be performed in full, unseparated blood prior separation of the cells.

RNA is isolated using the Rneasy® kit (Qiagen, Hamburg, Germany) following the instructions of the supplier and eluted in 100 µl H₂O. The RNA is precipitated with sodium acetate and Ethanol, washed with 70 % ethanol and resuspended in 11 µl H₂O.

The RNA is mixed with 4 µl dT₁₂ primers (Microsynth, Balgach, Switzerland) and incubated in a thermal cycler at 90° C for 2 minutes. The temperature is ramped to 42° C in 20 minutes. Following components are added: 6 µl 5× reverse first strand buffer (GIBCO BRL, Basel, Switzerland), 2 µl 0,1 M DTT (Life Technologies, Basel, Switzerland), 20 U Rnase inhibitor (Roche, Mannheim, Germany), 1 µl of dCTP, dGTP, dTTP (each 10 mM; Perkin Elmer), 5 µl [α-³³P]-dATP (0,4 MBq/µl; Hartmann, Braunschweig, Germany) and 1 µl RNase H-reverse transcriptase (Superscript II, Life Technologies). Alternatively, for example fluorescent nucleotides can be used in combination with e.g. glass arrays or any other solid, non-fluorescent carrier. The mixture is incubated at 42°C for 3 hours and filled up to 100 µl with H₂O. The cDNA is purified using silica-gel columns (QIAquick, Qiagen) and eluted in 100 µl H₂O. Again 1 µl are measured in a scintillation counter to estimate the percentage incorporation of labeled nucleotides in the cDNA.

The oligonucleotide probes and an array may be constructed as described above, e.g. the oligonucleotides listed in Figure 8 can be spotted on nylon membrane: The oligonucleotides are spotted on the membrane using a 96 slot manifold (Schleicher & Schuell, Dassel, Germany). The DNA is denatured in 50 µl 1N NaOH incubated for 5 min at 37°C and subsequently mixed with 12x SSC buffer (1.8 M NaCl, 0.18 trisodium citrate). A total amount of 10 ng of each oligonucleotide is added in a volume of 100 µl to allow equal distribution on the membrane and are arranged in duplicates. In the present example, however, a commercially available array membrane is used. One probe from patient blood and one probe from healthy volunteer blood is each hybridized on a human cytokine expression array membrane (R&D Systems, Abingdon, UK). Hybridization in the present example is performed as described in the human cytokine expression array handbook (R&D). The membranes are washed 20 minutes with 30 ml of a 0,1 × SSPE buffer (SSPE stock is 20x: 3.6M NaCl, 0.2 M NaH₂PO₄, 0.02 M EDTA, pH7.7), 1% (w/v) SDS solution. The arrays are exposed 24 hours to imager-screens (Fuji Film, Tokyo, Japan). The screens are analyzed using the FLA 3000 phosphor imager system (Fuji) and evaluated with the AIDA program (Raytest, Urdorf, Switzerland). To compare the intensity of spots containing different membranes housekeeping genes like GAPDH and L19 are normalized.

Out of 375 different genes 100 have been found to be regulated in either asthma or atopic dermatitis patients. Some of the genes with great amplitude are shown in Figure 2. The maximal amplitude detected was 40x, however in most cases the differences did not exceed a factor of 5. Although some genes were disregulated both in asthma and atopic dermatitis, the amplitude was in most cases different. Furthermore, genes with greatest changes were different between atopic dermatitis and asthma (Fig. 2). These results show that the inventive method described here allows the differentiation between allergic diseases.

An example of an so-called ATRP (activated CD4⁺ T cell mRNA profiling system, according to one embodiment of the inventive method) for differential diagnosis of allergic diseases is shown in Figure 2: A set of genes is measured by arrays or other systems. Fold differences are shown for each gene and the order and scale of each patient is the same, resulting in an individual profile for each patient. These profiles are now overlaid (middle panel) for all atopic dermatitis and for all asthma patients resulting in an (ideal) asthma or atopic dermatitis profile. It should be noted that this phenotype can be clinically more challenging such as revealing bronchial hyperreactivity, steroid resistance or yet unidentified subgroups of allergic disease.

The comparison of atopic dermatitis and asthma is shown in the right panel: Although some genes can be increased or decreased both in asthma or atopic dermatitis, some genes clearly appear only changed in asthma or atopic dermatitis. This comparison is shown in more detail in figures 3-7. Generally it can be concluded that group 1 genes (Fig. 3, surface activation markers) are more frequently increased in AD patients whereas asthma patients showed rather reduced levels. Group 1 genes will give information how active/acute inflammation is going on. For group 2 (Fig. 4) there is generally a higher expression in AD, whereas asthma patients are more suppressed in these genes. Of note HCC-4 is regulated to a lower degree and acts as a marker of reduced tolerogenic capacity of the patient. In contrast TRAIL R2 was increased in both patients reflecting alterations in cell death pathways. Group 3 (Fig. 5) is consistently downregulated in asthma patients. AD patients with decreased expression levels in this field possibly carry a risk to develop asthma, group 4 genes (Fig. 6) are yet not characterized in their function, but are generally decreased in asthma patients. The same atopic dermatitis patient which showed asthma indicative decreases in group 3 genes is also decreased in group 4 genes, confirming the asthma like profile. Group 5 genes (Fig. 7) are parameters, which are related to nerve growth and activation relevant for pain diagnosis and for bronchial hyperactivity. A consistent high expression occurs within the asthma patients. The NGF R is an exception, which was decreased in asthma and increased in AD patients. The complete description of phenotype relation of each gene is listed in the table of Figure 8.

As demonstrated above, mRNA of activated CD4⁺ T cells allows differential diagnosis of allergic diseases. The CD4⁺ T cells are isolated and stimulated under defined conditions in vitro. Subsequently, mRNA is subjected to multigene analysis such as DNA arrays. Gene expression profiles can be created, which allow on the basis of the activated T cell mRNA the prediction of certain phenotypes such as asthma or atopic dermatitis.

### References

1. Homburger, H.A. 1986. Diagnosis of allergy: in vitro testing. Crit Rev Clin Lab Sci 23, no. 4:279.
2. Sampson, H.A. 1999. Food allergy. Part 2: diagnosis and management. J Allergy Clin Immunol 103, no. 6:981.
3. Ono, S.J. 2000. Molecular genetics of allergic diseases. Annu Rev Immunol 18:347.
4. Werfel, T., A. Morita, M. Grewe, H. Renz, U. Wahn, J. Krutmann, and A. Kapp. 1996. Allergen specificity of skin-infiltrating T cells is not restricted to a type-2 cytokine pattern in chronic skin lesions of atopic dermatitis. J Invest Dermatol 107, no. 6:871.
5. Sager, N., A. Feldmann, G. Schilling, P. Kreitsch, and C. Neumann. 1992. House dust mite-specific T cells in the skin of subjects with atopic dermatitis: frequency and lymphokine profile in the allergen patch test [see comments]. J Allergy Clin Immunol 89, no. 4:801.
6. Horneff, G., C. Schou, and V. Wahn. 1996. Diagnostic significance of in vitro T cell proliferative responses to house-dust mite Der p 1 in children with dust-mite allergy. Allergy 51, no. 11:842.
7. Rasanen, L., and M.L. Tuomi. 1992. Diagnostic value of the lymphocyte proliferation test in nickel contact allergy and provocation in occupational coin dermatitis. Contact Dermatitis 27, no. 4:250.
8. Mtulsky, H. 1995. Intuitive Biostatistics. Oxford University Press

## Claims

1. Method for diagnosing the presence of or predisposition for (a) allergic disease(s) using expression profiling images comprising the steps of:
(a) *in vitro* activating lymphocytes or monocytes/macrophages obtained from whole blood of an individual to be diagnosed for the presence of or predisposition for (a) specific allergic disease(s),
(b) providing ligands selected from the group consisting of mRNA, oligonucleotides, cDNAs, proteins or functional fragments thereof, isolated or generated from said lymphocytes or monocytes/macrophages
(c) contacting said ligands with a set of immobilized receptors selected from the group consisting of oligonucleotide or cDNA probes and antibodies or functional fragments thereof, specifically representing a respective set of genes involved with the presence of or predisposition for (a) specific allergic disease(s) to be diagnosed, and qualitatively and quantitatively detecting the presence of bound ligand/receptor complexes to obtain an expression profiling image being representative for the current status of the individual to be diagnosed,
(d) comparing the expression profiling image obtained in step (c) with the expression profiling image(s) of normal individuals and/or with the expression profiling images(s) of individuals having a predisposition for or suffering from (a) specific allergic disease(s) to be diagnosed, and
(e) excluding or diagnosing (a) suspected allergic disease(s) or a predisposition therefor on the basis of the results of the comparison obtained in step (d).

2. Method according to claim 1, wherein in step (a) said lymphocytes are selected from the group consisting of CD4⁺ T cells and CD8⁺ T cells.

3. Method according to claims 1 or 2, wherein in step (a) said lymphocytes or monocytes/macrophages are activated by exposing the cells to mitogens such as anti-CD3 and/or anti-CD28 antibodies; or phorbol 12-myristate 13-acetate and ionomycin; or phytohemagglutinin and ionomycin; or staphylococcal enterotoxin B; or lipopolysaccharid; or a combination thereof.

4. Method according to any one of the preceding claims, wherein the allergic disease(s) or predisposition(s) to be diagnosed is/are selected from the group consisting of allergic asthma, allergic rhinitis, food allergies, anaphylactic shock risk, atopic dermatitis, immediate-type allergic reactions, and insect allergies.

5. Method according to claim 4 wherein the allergic disease(s) to be diagnosed is/are allergic asthma and atopic dermatitis.

6. Method according to any of the preceding claims, wherein in step (c) said set of receptors specifically representing a respective set of genes is selected from the group consisting of genes coding for EpCAM, DNAM, Endoglin, Flt-3/Flk-2 R, c-met, MPL R, MSP R, IL-18 R, Tie-2, fractalkine, HCC-4, I-309, Agouti-related transcript, PREF-1, SARP-3, Urokinase R, MMP-15, EBAF, TRAIL R2, amphiregulin, betacellulin, cripto, erbB1, erbB3, TGF-α, ephrin-A1, ephrin-A2, EphA1, EphA2, EphA3, EphA4, EphB4, EphB6, CNTF, GDNF, GFRα1, GFRα2, neurophilin-1, NGF R, ICAM-1, angiogenin, VEGF, VEGF-8, VEGF-D, CD34, SLAM, eolakin-2, IL-8, MIP-ld, MCP-4, MDC, midkine, TARC, Flt-3 ligand, G-CSF, GM-CSF, c-kit ligand, leptin, oncostatin M, osteopontin, PP14, SARP-1, follistatin, IGF binding protein, IGF binding protein, IGF binding protein, Epo R, G-CSF-R, IL-11 Ra, IL-15 Ra, eNOS, MMP-12, inhibin A (a subunit), 4-1BB, CD30, FasL, TRAIL R2, GM-CSF Rb, IFN-a/b Rb, IGF-I R, c-kit, M-CSF R, FGF acidic, FGF-3, FGF-4, FGF R2, FGF R3, integrin-a5, IL-2, IL-3, IL-4, IL-5, IL-6, IL-7, IL-10, IL-11, IL-1 RII, IL-2 Ra, IL-2 Rb, IL-2Rg; and allelic variants or mutants thereof.

7. Method according to claim 6, wherein said set of receptors is selected from oligonucleotide probes having a sequence defined by, or correlated to, or derived from the group of genes consisting of candidate genes indicated in the following list:
| Gene coding for | Startposition | lenght | Accession No |
|---|---|---|---|
| EpCAM | 1259 | 40 | X13425 |
| DNAM | 2369 | 40 | U56102 |
| Endoglin | 1878 | 40 | X72012 |
| Flt-3/Flk-2 R | 2367 | 40 | U02687 |
| c-met | 4305 | 40 | X54559 |
| MPL R | 1854 | 40 | M90102 |
| MSP R | 3950 | 40 | X70040 |
| IL-18 R | 2947 | 40 | U43672 |
| Tie-2 | 2618 | 42 | L06139 |
| Fractalkine | 216 | 40 | U91835 |
| HCC-4 | 988 | 40 | U91746 |
| I-309 | 427 | 40 | M57502 |
| Agouti-Related Transcript | 720 | 40 | U88063 |
| PREF-1 | 1395 | 40 | U15979 |
| SARP-3 | 1108 | 40 | AF017988 |
| Urokinase R | 120 | 40 | Z46797 |
| MMP-15 | 559 | 40 | D86331 |
| EBAF | 1847 | 42 | U81523 |
| TRAIL R2 | 2922 | 40 | AF016266 |
| Amphiregulin | 907 | 40 | M30704 |
| Betacellulin | 1029 | 38 | NM 001729 |
| Cripto | 1034 | 39 | X14253 |
| erbB1 | 4253 | 40 | X00588 |
| erbB3 | 4445 | 40 | M29366 |
| TGF-a | 692 | 40 | M31172 |
| Ephrin-A1 | 929 | 40 | M57730 |
| Ephrin-A2 | 197 | 40 | AJ007292 |
| EphA1 | 2598 | 40 | M18391 |
| EphA2 | 2920 | 40 | M59371 |
| EphA3 | 2058 | 40 | M83941 |
| EphA4 | 2443 | 42 | L36645 |
| EphB4 | 3163 | 40 | U07695 |
| EphB6 | 3276 | 41 | D83492 |
| CNTF | 682 | 42 | X60542 |
| GDNF | 379 | 40 | L19063 |
| GFRalphal | 942 | 40 | U97144 |
| GFRalpha2 | 438 | 40 | AF002700 |
| Neuropilin-1 | 2490 | 40 | AF018956 |
| NGF R | 2885 | 40 | M14764 |

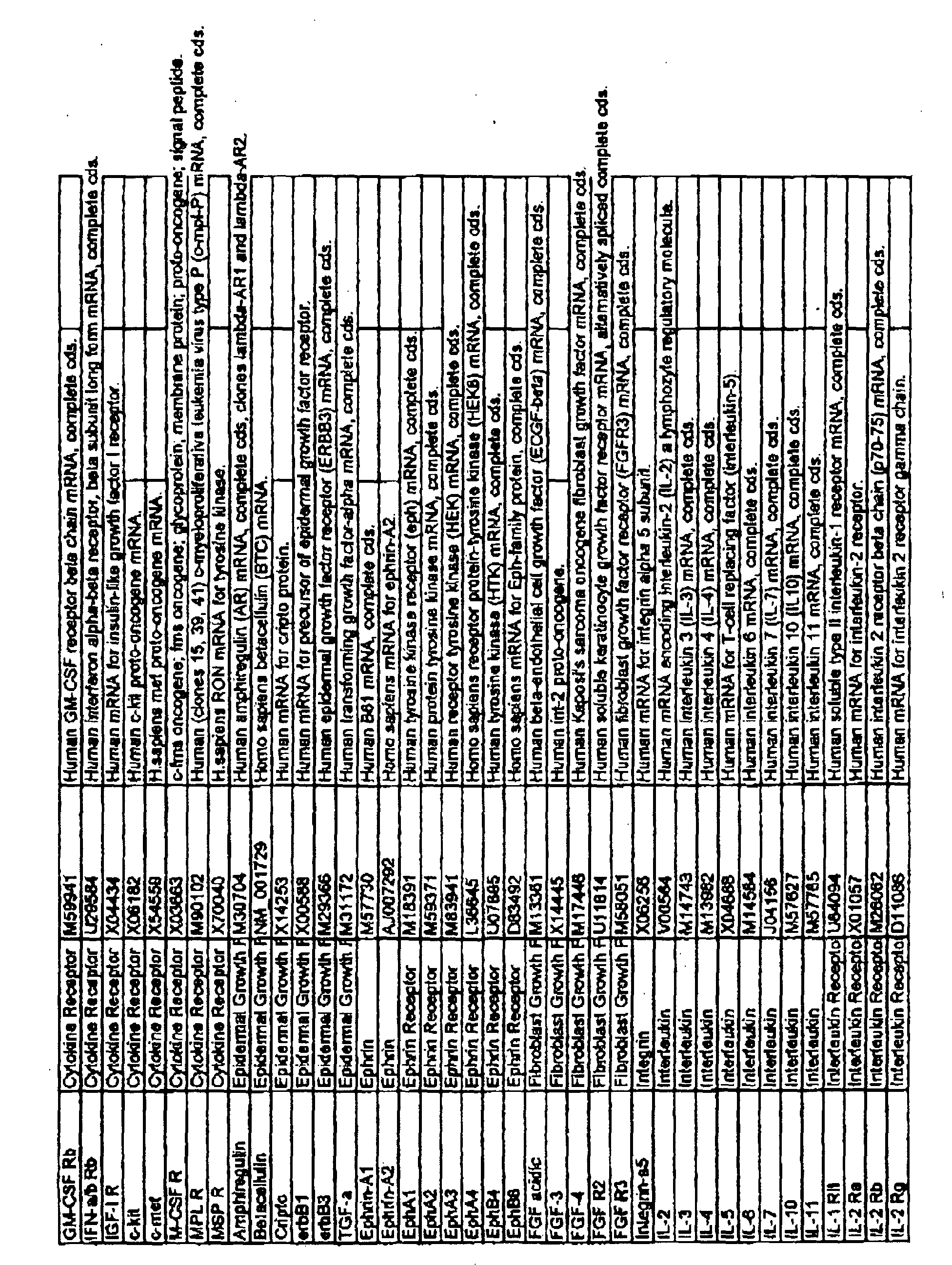

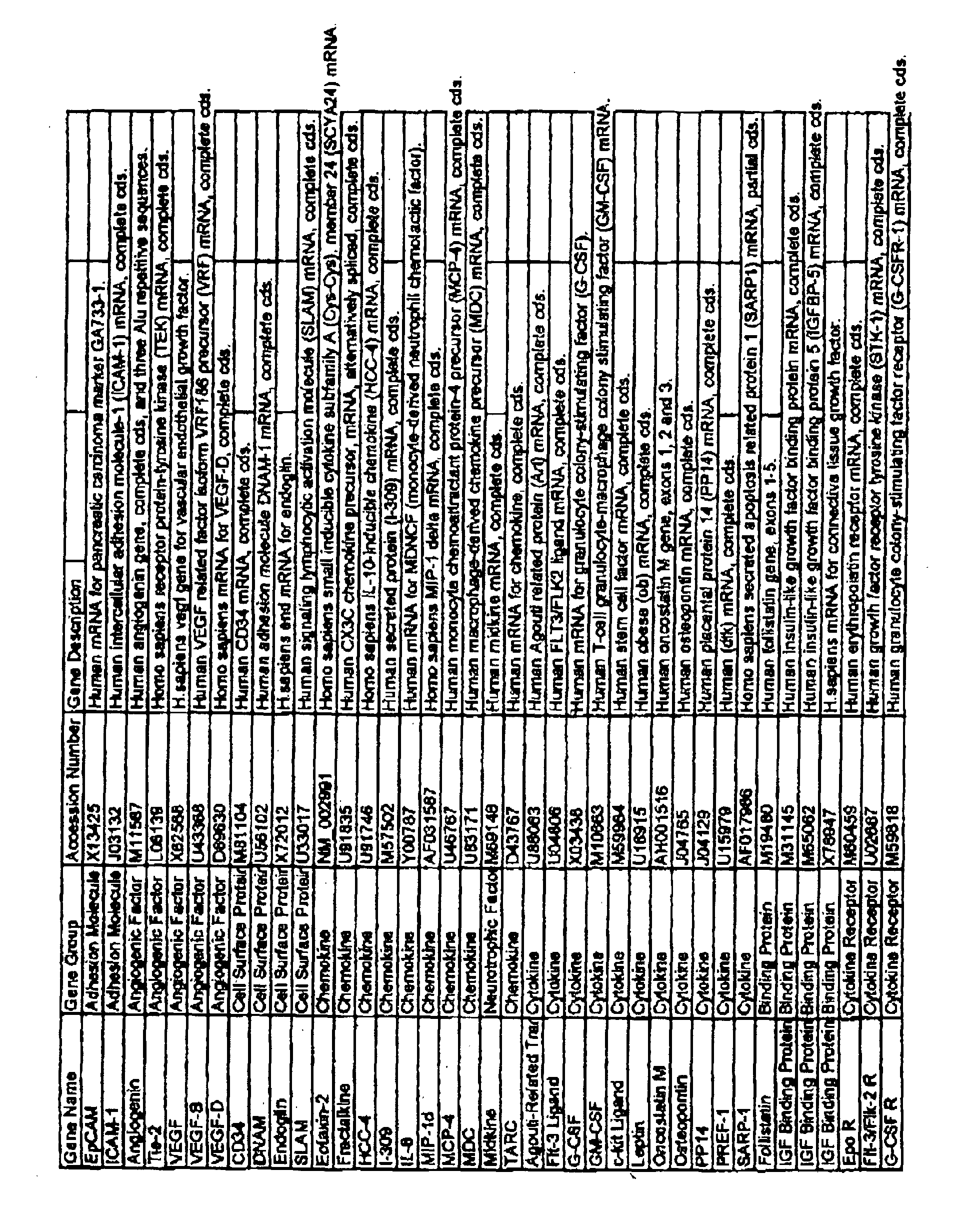

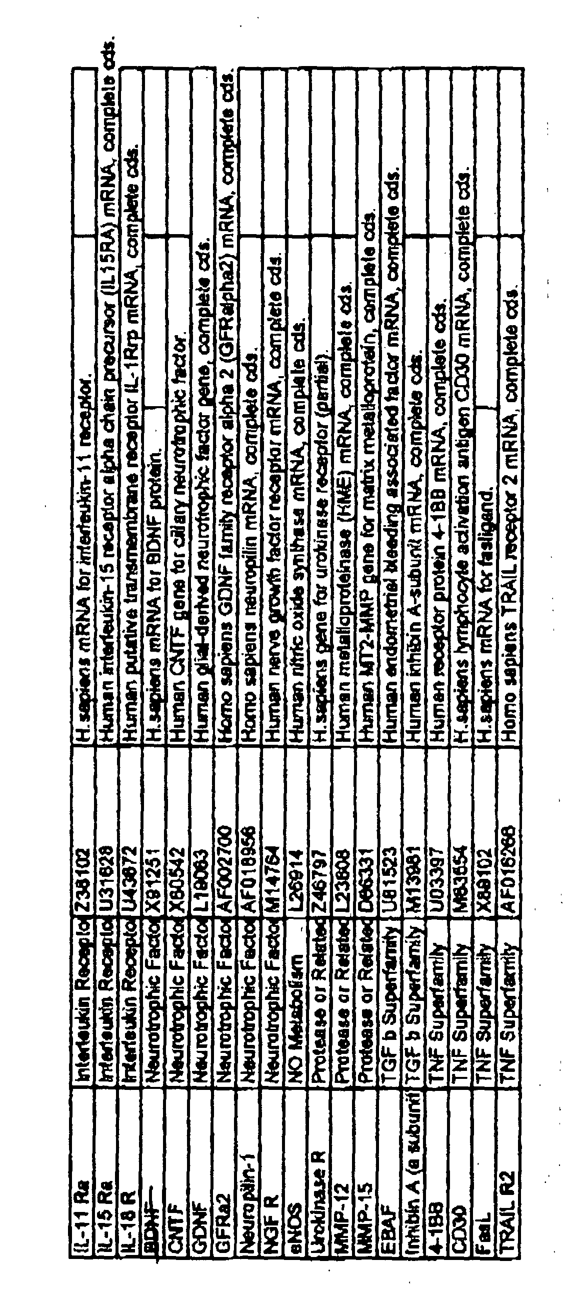
,wherein each oligonucleotide probe is obtainable on the basis of structural information mediated by the sequence data provided by the respective accession number set out for each candidate gene.

8. Method according to any one of the preceding claims, wherein in step (c) said immobilized receptors are located on a surface.

9. Method according to claim 8, wherein said immobilized receptors form a patterned array on said surface.

10. Screening assay for evaluating the allergenic and/or anti-allergical potential of (an) analyte substance(s) using expression profiling images comprising the steps of:
(a) *in vitro* activating lymphocytes or monocytes/macrophages, preferably being standardized to represent a baseline expression profiling image characteristic for a normal's status,
(b) incubating said activated lymphocytes or monocytes/macrophages with (an) analyte substance(s) to be evaluated for its (their) allergenic and/or anti-allergical potential,
(c) providing ligands selected from the group consisting of mRNA, oligonucleotides, cDNAs, proteins or functional fragments thereof, isolated or generated from said lymphocytes or monocytes/macrophages,
(d) contacting said ligands with a set of immobilized receptors selected from the group consisting of oligonucleotide or cDNA probes and antibodies or functional fragments thereof, specifically representing a respective set of genes involved with the presence of or predisposition for (a) specific allergic disease(s) to be diagnosed, and qualitatively and quantitatively detecting the presence of bound ligand/receptor-complexes to obtain an expression profiling image being representative for the allergenic and/or anti-allergical potential of the analyte substance(s) to be evaluated,
(e) evaluating the allergenic and/or anti-allergical potential of the analyte substance(s) by comparing the expression profiling image obtained in step (d) with the expression profiling image generated from normal individuals, or preferably by directly analyzing the expression profiling image obtained in step (d).

11. Diagnostic tool suitable for use in a method according to any one of claims 1 to 9 or 10 comprising on its surface a set of immobilized receptors selected from the group consisting of oligonucleotide or cDNA probes and antibodies or functional fragments thereof, capable of specifically binding ligands selected from the group consisting of mRNA, oligonucleotides, cDNAs, proteins or functional fragments thereof, representing a respective set of genes involved with (a) specific allergic disease(s) or a predispositon therefor to be diagnosed.

12. Diagnostic tool according to claim 11, wherein said immobilized receptors form a patterned array on said surface.
